# EUROPEAN PATENT SPECIFICATION

(11) **EP 3 365 032 B1**
(45) Date of publication and mention of the grant of the patent: **13.03.2024**
(21) Application number: 16858222.9
(22) Date of filing: 20.10.2016
(51) Int. Cl.: A61L 9/20, A61F 7/00

(54) **UV STERILIZATION SYSTEM FOR FORCED-AIR PATIENT HEATING SYSTEMS**
UV-STERILISATION SYSTEM FÜR PATIENTENHEIZSYSTEME MIT ZWANGSBELÜFTUNG
SYSTÈME DE STÉRILISATION PAR UV POUR SYSTÈMES DE CHAUFFAGE À AIR FORCÉ À DESTINATION DE PATIENTS

(30) Priority: 23.10.2015 US 201514921638
(43) Date of publication of application: 29.08.2018
(73) Proprietor: Cleanco Bioscience Group, LLC, Jupiter, Florida 33458 (US)
(72) Inventor: KROSNEY, Mark D., Port Saint Lucie, Florida 34952 (US)
(74) Representative: Kompter, Hans-Michael
(86) International application number: PCT/US2016/057932
(87) International publication number: WO 2017/070359

(56) References cited:
- EP-A1- 2 554 583
- EP-A1- 2 921 183
- WO-A1-2010/071814
- US-A- 5 225 167
- US-A- 5 761 908
- US-A- 5 964 792
- US-A- 6 039 926
- US-A- 6 053 968
- US-A1- 2005 163 648
- US-A1- 2009 004 047
- US-A1- 2012 168 641
- US-A1- 2014 030 144
- US-A1- 2014 271 374
- US-A1- 2014 348 701

## Description

### FIELD OF THE INVENTION

This invention relates generally to air sterilization and disinfection, and more particularly to a system for sterilizing and disinfecting air in forced-air patient heating systems.

### BACKGROUND

According to the Centers for Disease Control, there are over 51 million surgeries performed in the United States alone each year [Centers for Disease Control and Prevention Online FASTSTATS - Inpatient Surgery]. The majority of these surgeries require advanced techniques for regulating the patient's core body temperature. It is known in the art that a typical operating room is kept at about 20°C, thereby making it difficult to keep a patient's core body temperature between the desired 36-38°C range. To further complicate matters, general anesthesia accelerates temperature loss from a patient. Without supplemental warming in this environment, a patient's core body temperature would quickly drop below 35°C to a hypothermic state. Persons having ordinary skill in the art will appreciate that this may lead to serious complications such as increased incidents of blood clots, wound infection, and cardiac arrest. For these reasons, it is important to maintain a patient's normal body temperature during surgery. It is well known in the art to utilize a warming device in order to maintain the patient's normal body temperature.

There are multiple devices known in the art that are used to warm a patient in an operating room during and after a surgical procedure. Common methods of patient warming include passive warming, such as through the use of insulators, and active heating, through the use of convection or conduction based devices. One of the most common warming methods is known in the art as forced-air convection [Mahoney CB, and Odom, J. "Maintaining intraoperative normothermia: a meta-analysis of outcomes with costs"]. Forced-air convection systems are well-described in the prior art and typically use a pump and heater system to blow warm air through a flexible hose and into an inflatable blanket, gown, or other covering in contact with the patient. The covering is typically inflated by the introduction of the forced-air through an inlet. An aperture array on the underside (patient-side) of the covering exhausts the heated air directly to the patient's body, thereby creating an ambient environment around the patient, the characteristics of which are determined by the temperature of the thermally-controlled forced air, which has the effect of raising the patient's body temperature through this forced-air convection.

While the prior-art forced-air convection warming systems have achieved their objective of regulating patient temperature, they have also brought with them serious, and undesirable, side-effects.

Studies, such as those reported in "Convection warmers - not just hot air," by Avidan, Jones, Ing, Khoosal, Lundgren, and Morrell, have indicated that forced-air convection warming systems are a potential source for nosocomial infection. Nosocomial infections are infections that have been caught in a hospital and are potentially caused by organisms that are resistant to antibiotics. A nosocomial infection is specifically one that was not present or incubating prior to the patient's being admitted to the hospital, but occurring within 72 hours after admittance to the hospital.

Other studies such as those reported by Albrecht, Gauthier, and Leaper, in "Forced-air warming: a source of airborne contamination in the operating room?" found that forced-air warming systems have the potential to generate and mobilize airborne contamination in the operating room. The design of forced-air warming blowers was found to be questionable for preventing the build-up of internal contamination and the emission of airborne contamination into the operating room. A significant percentage of forced-air warming blowers with positive microbial cultures were emitting internally generated airborne contamination within the size range of free floating bacteria and fungi (<4 µm) that could, conceivably, settle onto the surgical site.

Although forced air warming systems are the preferred method of patient warming, the design of the current state of the art warmers have inherent design flaws that contribute directly to the delivery of airborne pathogens to the patient. Current air warmers are small. Their compact size is intentional in order to not be obtrusive in the operating suite, as well as portable to go with the patient from room to room. These small units are more often than not hung off the side or foot of the patient bed. This location places the air intake of the unit closer to the floor, and most importantly, outside of the sterile field.

Pathogen laden air is drawn in to the unit, where it then passes over a heating element, and is then expelled through a hose to the patient location, typically through the use of a blanket or covering, as described above. While most of these units incorporate a particulate filter, these filters do not keep out most pathogens, and their effectiveness depends directly on the care and maintenance of the unit. Like all particulate filters, they need to be regularly cleaned and/or replaced.

Once the pathogens have entered the unit, the heating chamber creates a breeding ground for fungi and other pathogens that thrive in warm, dark environments.

All of this combines to create a direct path for pathogen-laden air to be introduced directly into the sterile field - and directly to the patient.

There is a growing demand for improvements in hospital settings to reduce the transmission of pathogens. This demand is driven by hospitals that have to deal with an increasing amount of cases of infections, not caused by the patient's diagnosis upon admission, but rather, due to airborne pathogens that exist in a hospital environment. Highly effective devices and methods of removing airborne pathogens using UV LEDs are disclosed in U.S. Pat. No. 8,900,519, as well as the other priority documents,

It would, therefore, be desirable and beneficial to have an apparatus, and related system and method that purifies and sterilizes air before warming the air and distributing the heated air to the patient. Furthermore, it would be desirable to have an air sterilization and purification device that is compact, quiet, and unobtrusive, while also being highly effective in the removal and/or neutralization of harmful airborne pathogens in a forced-air warming system for patient temperature control.

The present invention is unique when compared with other known devices because the present invention provides: (1) a compact footprint; (2) effective pathogen removal directly at the site of the blower/warmer; and (3) ease of maintenance.

The present invention is unique in that it is structurally different from other known devices or solutions. More specifically, the present invention is unique due to the presence of: (1) an airflow and irradiation management chamber comprising a single or a plurality of turbulators; (2) UV LEDs embedded in the walls of the airflow and irradiation management chamber; (3) one or more high efficiency particulate filters and/or HEPA filters; and (4) a blower/warmer that heats and delivers thermally controlled air that has been sterilized and, effectively, pathogen-free. Patent publications EP2921183A1 and US2014/271374A1 are relevant for the present invention.

### SUMMARY OF THE INVENTION

The invention is defined in appended independent claim 1. Further embodiments are defined in appended dependent claims.

The present invention discloses an improvement to the UV sterilization and disinfection devices disclosed in the priority documents, and relates to system.

With respect to the system , embodiments include a compact, highly effective air sterilization and disinfection apparatus, which delivers clean, pure air directly into a blower/warmer module for clean and effective management of patient body temperature.

In a preferred embodiment, the system combines wavelength-specific, high-output UV LEDs with an airflow and irradiation management chamber that facilitates the necessary UV dosage by increasing the dwell time of the airflow being treated. This system can be used in hospitals, clinics, operating rooms, and other environments where it is desired to deliver clean, pure air directly into a blower/warmer module for clean and effective management of patient body temperature. The compact, quiet, and unobtrusive nature of this system makes it particularly well suited for use in surgical environments.

Generally, the system comprises an electronics and control module, a means of drawing room air into, and expelling from, the apparatus, a heater, an air management chamber, an array of wavelength-specific, high-output UV LEDs, a particulate filter means, and a housing.

With respect to the particulate filter means, said filter may be chosen from various materials known in the art to filter airborne particles such as high efficiency particulate filters and HEPA filters.

With respect to the system it should be further noted that the selection of the wavelength of the UV LEDs as well as the design of the airflow and irradiation management chamber is critical in order to manage the level and duration of UV light dosage in order to effectively sanitize the incoming air.

Generally, the steps to carry out a method associated with the system are comprised of:
drawing air into the apparatus;
exposing the air to sufficient UV radiation to achieve at least a 2 log (99%) kill rate;
heating the now sterilized air; and
expelling the now heated and sterilized air to the patient, whereby the system is used to deliver clean, pure air to the patient for clean and effective management of patient body temperature.

### BRIEF DESCRIPTION OF THE DRAWINGS

These and other features, aspects, and advantages of the present invention will become better understood with regard to the following description, appended claims, and accompanying drawings where:
FIG. 1 shows a simplified block diagram representation of an embodiment of the invention, as shown;
FIG. 2 shows a perspective view of an embodiment of the invention, as shown;
FIG. 3 shows a composite view of an embodiment of the invention, including a cross-sectional view of an embodiment of an airflow and irradiation management chamber, as shown; and
FIG. 4 shows a composite view of an embodiment of the invention, including perspective, orthographic projection, and cross-sectional views of an apparatus for sterilizing and disinfecting air.

### DETAILED DESCRIPTION OF THE DRAWINGS

In the Summary of the Invention above and in the Detailed Description of the Drawings, and the claims below, and in the accompanying drawings, reference is made to particular features of the invention. It is to be understood that the disclosure of the invention in this specification includes all possible combinations of such particular features. For example, where a particular feature is disclosed in the context of a particular aspect or embodiment of the invention, or a particular claim, that feature can also be used, to the extent possible, in combination with and/or in the context of other particular aspects and embodiments of the invention, and in the invention generally , within the scope of the appended claims.

The term "comprises" and grammatical equivalents thereof are used herein to mean that other components, ingredients, steps, etc. are optionally present. For example, an article "comprising" (or "which comprises") components A, B, and C can consist of (i.e., contain only) components A, B, and C, or can contain not only components A, B, and C but also one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

The term "at least" followed by a number is used herein to denote the start of a range beginning with that number (which may be a range having an upper limit or no upper limit, depending on the variable being defined). For example "at least 1" means 1 or more than 1. The term "at most" followed by a number is used herein to denote the end of a range ending with that number (which may be a range having 1 or 0 as its lower limit, or a range having no lower limit, depending upon the variable being defined). For example, "at most 4" means 4 or less than 4, and "at most 40%" means 40% or less than 40%. When, in this specification, a range is given as "(a first number) to (a second number)" or "(a first number)-(a second number)," this means a range whose lower limit is the first number and whose upper limit is the second number. For example, 25 to 100 mm means a range whose lower limit is 25 mm, and whose upper limit is 100 mm.

While the specification concludes with claims defining the features of embodiments of the invention that are regarded as novel, it is believed that the invention will be better understood from a consideration of the following description in conjunction with the figures, in which like reference numerals are carried forward.

### The Apparatus

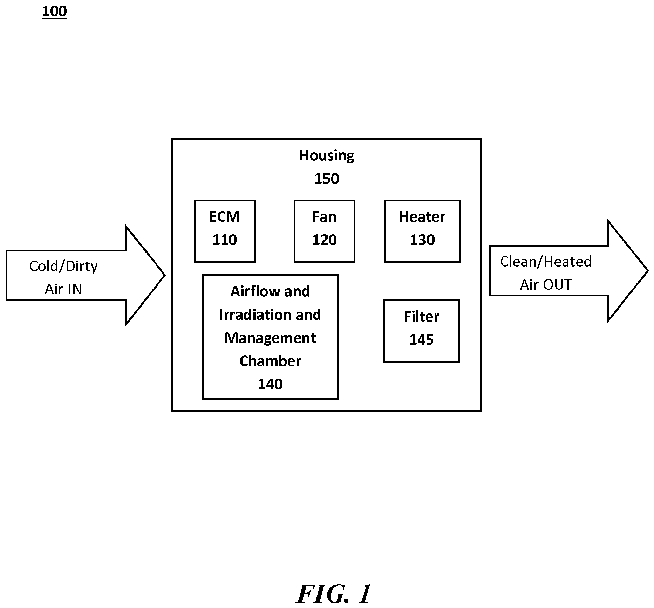

One embodiment, in the form of a convective air warming, sterilization and disinfection apparatus 100, as shown generally in the figures and particularly in FIG. 1, can comprise: an electronics and control module 110; a fan 120; a heater 130; an airflow and irradiation and management chamber 140; a filter 145; and a housing 150 whereby the apparatus is capable of achieving at least a 2 LOG kill of airborne pathogens, warming the sterilized air, and delivering it to the patient.

This apparatus can be used in hospitals, clinics, operating rooms, and other environments where it is desired to deliver clean, pure air directly into a blower/warmer module for clean and effective management of patient body temperature. The compact, quiet, and unobtrusive nature of this apparatus makes it particularly well suited for use in surgical environments.

For simplicity of disclosure, certain components of the apparatus are described here in general terms as the specifics of the component would be known to one having ordinary skill in the art. For example, the electronics and control module 110 may comprise various sub-components and features as would be necessary to provide and regulate power to the various parts of the apparatus, receive input from a user, and provide feedback to a user. The fan 120 may be chosen among any of the various means of producing an airflow as is known in the art. Similarly, the heater 130 may be chosen among any of the various means of heating air as is known in the art. The filter 145, is a high efficiency particulate filtration means as is known in the art, for example, a HEPA filter.

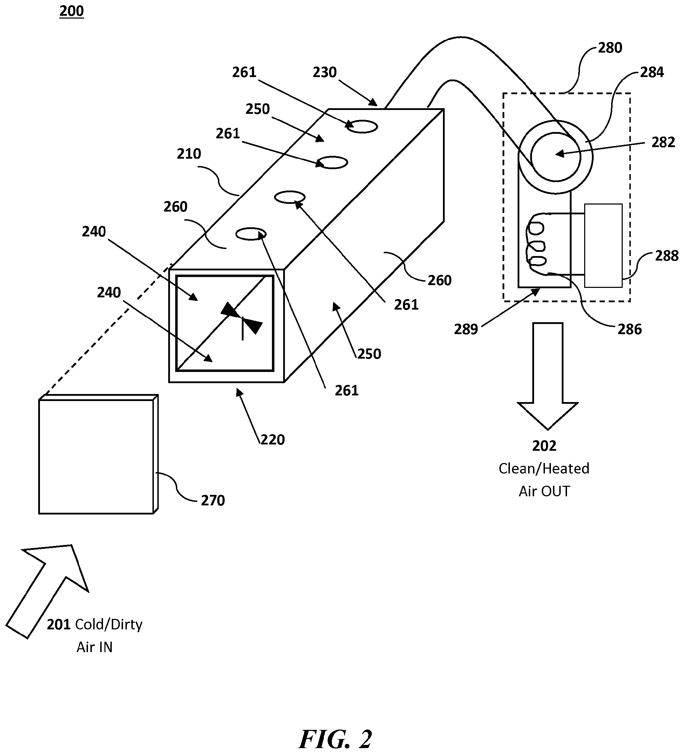

Referring now to the figures in general and FIG. 2 in particular, another embodiment of the present invention discloses an apparatus for delivering pathogen-reduced air in a forced-air patient heating system 200, the apparatus comprising: an airflow and irradiation management chamber 210 that creates a turbulent flow such that airborne pathogens are exposed to a dosage of UV radiation sufficient to penetrate and kill the pathogens, comprising: an inlet 220, an outlet 230, an inner surface 240, an outer surface 250, and a wall 260 bounded by the inner surface and the outer surface; a high efficiency particulate filter 270 operatively coupled to the airflow and irradiation management chamber inlet; and a heater/blower assembly 280 comprising: an inlet 282 operatively connected to the airflow and irradiation management chamber outlet 230, a fan 284, a heater 286, an electronics and control module 288, and an outlet 289.

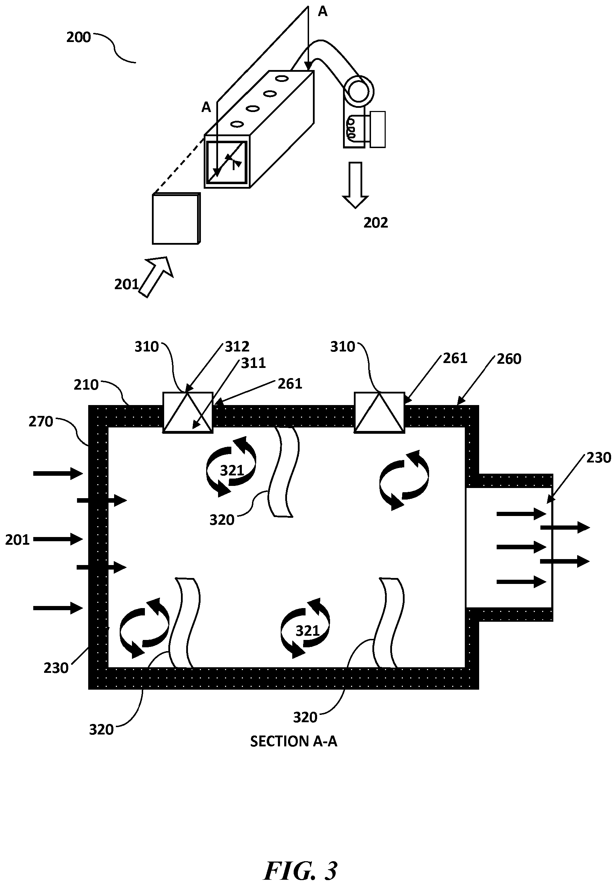

Referring now to Figures 2 and 3, another embodiment of the present invention discloses an apparatus 200 for delivering forced air to a patient temperature control system by warming, sterilizing, and disinfecting air, the apparatus comprising: an ultra-violet (UV) light blocking structure 210, configured to receive an air flow with a one or more airborne pathogens 201, said UV light blocking structure comprising an inlet 220, an outlet 230, a bounding surface 260 between the inlet and the outlet defining an inner area and an outer area, and a one or more aperture 261 through the bounding surface between the inner area and the outer area; a one or more UV light emitting diode (LED) 310 with an emitter portion 311 and a non-emitter portion 312, insertedly related to the one or more aperture such that the emitter portion is oriented toward the inner area of the UV light blocking structure and further inserted with sealing means as is known in the art to ensure that UV light does not escape through the aperture; a one or more turbulator 320 located within said inner area of the UV light blocking structure; a high efficiency particulate filter 270 operatively connected to the inlet 220 of the UV light blocking structure 210; and a heater/blower assembly 280 comprising: an inlet 282 operatively connected to the UV light blocking structure outlet 230; a fan 284; a heater 286; an electronics and control module 288; and an outlet 289 whereby the apparatus expels clean heated air 202.

The turbulators 320 disclosed in this embodiment, and throughout the disclosure, are physical structures designed to create turbulance. More specifically, the turbulators interact with an airflow, converting a laminar flow into a turbulent flow 321. One having ordinary skill in the art would recognize that the turbulators may be chosen from various configurations including, but not limited to, vanes, airfoils, and v-gutters.

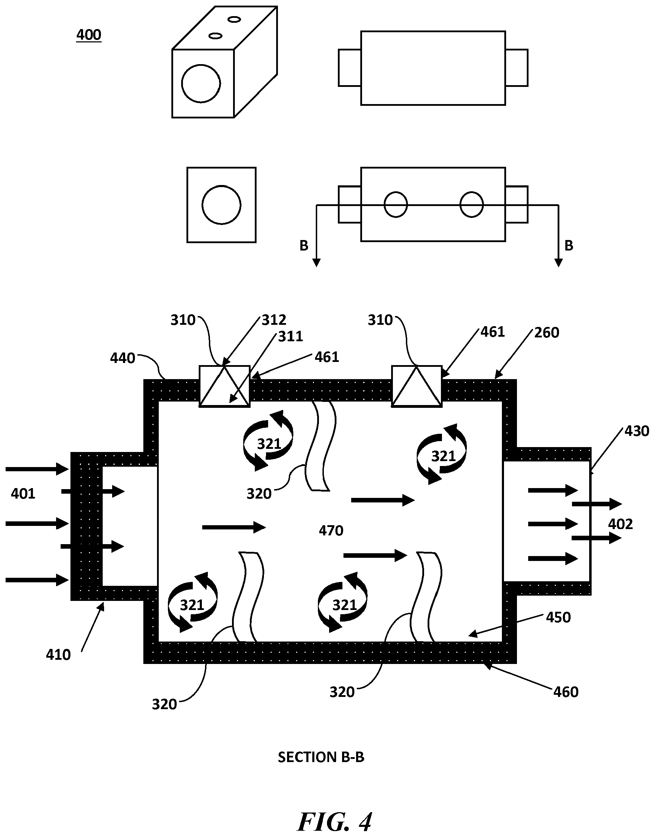

We refer now to FIG. 4 where we discuss another embodiment of the present invention disclosing an apparatus 400 for sterilizing and disinfecting air, the apparatus comprising: an inlet portion 410 configured to receive an air flow 401; a high efficiency filter 420 operatively coupled to the inlet portion 410; an outlet portion 430 configured to expell the now sterilized air flow 402; a non-ultraviolet (UV) light transmissive surface portion 440, comprising a UV light reflective inner surface 450, an outer surface 460, a one or more turbulator 320, and a one or more aperture, said non-UV light transmissive surface defining a substantially enclosed area 470 between the inlet portion and the outlet portion through which said air flow passes; and a one or more UV light emitting diode (LED) 310 inserted into said one or more apertures 461 such that a UV radiation is emitted into said enclosed area 470 thereby exposing said air flow to said UV radiation.

Another embodiment of the present invention discloses a kit for retrofitting an existing forced-air convection heater device for providing clean, thermally-controlled air to patients for the prevention of hypothermia as might occur intraoperatively or postoperatively. Embodiments of the kit may comprise: an ultra-violet (UV) light blocking structure, configured to receive an air flow with a one or more airborne pathogens, said UV light blocking structure comprising an inlet, an outlet, a bounding surface between the inlet and the outlet defining an inner area and an outer area, and a one or more aperture through the bounding surface between the inner area and the outer area; a one or more UV light emitting diode (LED) with an emitter portion and a non-emitter portion, insertedly related to the one or more aperture such that the emitter portion is oriented toward the inner area of the UV light blocking structure; a one or more turbulator located within said inner area of the UV light blocking structure; a high efficiency particulate filter located at the inlet of the UV light blocking structure; a hardware kit; and a housing. The hardware kit is configured to contain all necessary mechanical and electrical hardware components required to install the kit onto the existing device. The housing is configured to enclose all of the kit components and further comprises mounting and attachment interfaces so that, once installed, the kit forms a complimentary structure to the existing device.

In some embodiments of the present invention, the lower housing of the existing warmer/blower device is removed and discarded or recycled. The kit embodiment described above would then be installed such that the outlet of the airflow and irradiation management chamber (also described here as the ultra-violet light blocking structure) is sealably coupled to the inlet of the existing device's heater portion, the UV LED's are electrically connected to the electrical supply of the device, and the kit housing is mechanically attached to the existing device's housing using the mechanical and electrical components of the kit's hardware kit.

Another embodiment of the present invention is disclosed herein as an apparatus configured to be attached to, and accept an airflow from, the distal end of a hose, which is attached at the hose's proximal end to an airflow outlet of a convective air warming device. Since there may be situations where it is not desirable, or possible, to retrofit an existing warmer/blower device, UV sterilization of the warmed air *prior* to reaching the patient may be accomplished by the use of this embodiment of the present invention which comprises: an airflow and irradiation management chamber as described herein with an inlet and an outlet; and a housing.

The apparatus may further comprise a first adaptor means for sealably interfacing the warmer/blower device hose distal end to the airflow and irradiation management chamber's inlet, as well as a second adaptor means for sealably interfacing the airflow management chamber's outlet with the inlet of a patient warming blanket or other covering configured for convective air patient warming. The first and second adaptor means include various mechanical interfaces as will be readily appreciated by one having skill in the art. Male-Female couplings, gaskets, reducers, expanders, and clamps are all examples of adaptors that may be chosen as the first and second adaptor means.

In this embodiment, the UV LEDs of the airflow and irradiation management chamber will need to be powered separately from the warmer/blower device. As such, it is contemplated to be within the scope of this embodiment of the present invention that the apparatus may further comprise a power supply and regulation means. This power supply and regulation means may include, but is not limited to, a power plug, a voltage regulator, a transformer, a circuit breaker, and circuitry for powering, monitoring, and regulating the UV LEDs.

An alternative means of powering the UV LEDs in this embodiment may comprise a rechargeable battery pack. This rechargeable battery pack would be electrically connected to the system in order to provide power to the UV LEDs and any additional circuitry. The battery pack may be recharged by conventional charging means, as is known in the art, or, alternatively, it could be recharged by electricity generated by the rotational motion of turbines placed within the airflow and irradiation management chamber. The airflow current expelled from the warmer/blower system and passing through the airflow and irradiation management chamber would flow past the vanes of the turbines causing them to rotate. These turbines may be utilized in concert with, or instead of, turbulators as described above, creating a turbulent flow within the airflow and irradiation management chamber, and also generating an electrical current which is fed back to a charging circuit means in order to recharge the batter pack.

It is well known in the art that the warmer/blower device in patient warming systems is located at some distance from the patient. In these cases, the warmed air is delivered to the patient (typically to a blanket or other covering means) via a hose. In embodiments of the present invention where the UV sterilization of the air is accomplished at the distal (patient) end of the hose, that embodiment of the invention may further comprise a hose that replaces the existing device's hose. Said replacement hose would comprise means as is know in the art for interfacing with the existing warmer/blower as well as to the US sterilization device. The replacement hose would further comprise integral conductor means for connecting the UV sterilization device to power source. It would be clear to one having ordinary skill in the art that such integral conductor means would include such components as multiple insulated conductors integrally molded into the wall of the replacement hose with electrical connection means on each end.

It is contemplated to be within the scope of the present invention that seals, gaskets, baffles, and other light blocking means as is known in the art are implemented throughout the invention in order to prevent UV light from escaping the apparatus.

Embodiments of the invention disclosed herein may further comprise safety interlock means so that if any part of the system were to become open, exposing the UV LEDs, then the system would shut off the UV LEDs or the unit entirely so as to protect the user from exposure to UV light, electricity, and/or moving parts. Safety interlock means may include various solutions known in the art including, but not limited to, relays, contact closures, and circuit breakers.

Embodiments of the invention disclosed herein may further comprise a timer means to indicate to a user when it is time to replace the filters. Timer means may include various solutions known in the art including, but not limited to, processors and circuitry configured to notify the user via a visual indicator after a predetermined time of operation has elapsed.

It would be clear to one skilled in the art, as well as within the scope and intention of this disclosure, that while the above embodiment has been described as a UV sterilization apparatus connected to clean the airflow between the warmer/blower hose and the inflatable blanket/covering, it may equivalently be installed in between the warmer/blower device and the warmer/blower hose to the same effect.

It would be clear to one skilled in the art that, while the components of the embodiment are described here in a particular configuration or "order", it is still contemplated to be within the scope of the present invention to configure the components in a different "order" and still achieve the same invention. For example, an embodiment of the present invention may comprise an air flow that first passes through a filter, then through the airflow and irradiation management chamber, then into the heater, then expelled out of the unit through the action of a fan or compressor. Alternatively, the order of those components may be changed such that the airflow first passes through a filter, then through a fan or compressor, then into an airflow and irradiation management chamber, then into a heater, and then out of the unit.

Furthermore, embodiments disclosed and discussed here are intended to encompass the UV sterilization of air in conjunction with a warmer/blower device, where the UV sterilization device sterilizes air prior to entering the warmer/blower, between the warmer/blower and the output hose, or at the end of the output hose.

Furthermore, embodiments may comprise one or more than one of any component. For example, in addition to the embodiment described above, an embodiment may comprise a first filter at the unit inlet, a first fan at the unit inlet, a one or more airflow and irradiation management chambers, a second filter at the outlet of the one or more airflow and irradiation management chambers, a second fan between the one or more airflow and irradiation management chambers and a one or more heaters, a third fan between the one or more heaters and the unit outlet, and a third filter at the unit outlet.

The Airflow and Irradiation Management Chamber (see, generally, 140, 210, and 400)

Building upon the teachings disclosed in the priority documents, which have been incorporated by reference herein, we now discuss an airflow and irradiation management chamber. Commercially, the airflow and irradiation management chamber may be known as a STERITUBE^{™} or a STERIDUCT^{™}.

Referring to the figures in general, and to FIG. 4 in particular, the airflow and irradiation management chamber 400 is comprised of a hollow cross sectional area which is extruded to a desired length such as to define an inner surface 450, an outer surface 460 an inlet 410 and an outlet 430.

An embodiment of the airflow and irradiation management chamber may comprise a cross-sectional area that is substantially consistent throughout the length of the airflow and irradiation management chamber.

A further embodiment of the airflow and irradiation management chamber may comprise a cross-sectional area that varies in shape and/or size throughout the length of the airflow and irradiation management chamber.

The cross section of the one or more airflow and irradiation management chamber may be circular, elliptical, rectangular, or any other shape as may be chosen to maximize the airflow through the desired package size. Each airflow and irradiation management chamber is designed to sustain a specific volumetric throughput.

Embodiments of the airflow and irradiation management chamber may be substantially straight, substantially curved, or comprised of a combination of substantially straight and curved sections.

The airflow and irradiation management chamber itself may be manufactured utilizing various methods and materials as may be known in the art including, but not limited to, extruded plastics, formed metals, or a combination of materials.

Embodiments of the airflow and irradiation management chamber may further comprise a surface treatment on the inner surface 450 that provides for a diffuse reflection of the UV light. The use of diffuse reflectors increases the efficiency of the UV irradiation field by scattering the UV light rays, as opposed to specular reflective surfaces (such as polished metals) that merely reflect the UV ray at an angle equal to the angle at which the ray hits the surface. This diffuse reflection may be accomplished through a micro-texture, a coating, or a laminated material, such as polytetrafluoroethylene (PTFE).

Embodiments of the airflow and irradiation management chamber further comprise a one or more turbulators 320 located within the hollow section of the airflow and irradiation management chamber. Turbulators disrupt the airflow, by changing laminar airflow 401 into a turbulent airflow 321, thus ensuring that the airborne pathogens remain exposed to UV radiation for a sufficient amount of time such that the radiation can kill the pathogen. Turbulators may be chosen from various forms known in the art, including, but not limited to, turbine vanes, airfoils, v-gutters, grooves, ridges, and baffles.

The wall of the airflow and irradiation management chamber 260, that area bounded by the inner surface 450 and the outer surface 460, comprises a material and/or surface finish, that blocks UV light from passing through the wall. The airflow and irradiation management chamber is not, as may otherwise be known in the art, a "light pipe", "light conduit", or other means of transmitting UV light through any means of internal reflection or refraction. Embodiments of the airflow and irradiation management chamber comprise one or more apertures 461 creating openings in the airflow and irradiation management chamber wall configured to accept one or more UV LEDs. As discussed above, the each UV LED is sealed, using a sealing means as is known in the art, to the aperture so that no UV light may escape.

### The UV LEDs 312

The efficacy of UV light, especially in the "germicidal" spectrum, for the killing of pathogens is well known in the art. UV LEDs, specifically, are well-disclosed in the priority documents and, for brevity, will not be further discussed here. UV LEDs are chosen for this apparatus because of their size, power, and long life. The UV LEDs are selected based upon the desired wavelength and power rating. The number and distribution of these UV LEDs in the airflow and irradiation management chamber are to be such as to maximize the radiant flux within each airflow and irradiation management chamber.

Embodiments of the present invention comprise a one or more UV LEDs sealably assembled into the one or more apertures in the airflow and irradiation management chamber wall such that the one or more UV LEDs emit UV light into the interior of the airflow and irradiation management chamber, namely, that area defined and enclosed by the inner surface of the airflow and irradiation management chamber through where the pathogen laden airflow passes between the airflow and irradiation management chamber inlet and the airflow and irradiation management chamber outlet.

The one or more UV LEDs are electrically connected to the electronics and control module.

### The Heater (see, generally, 130 and 286)

Embodiments of the present invention may further comprise means for heating the airflow. This heating means may be accomplished by any of various methods known in the art, for example, by introducing a current to a length of wire with a resistance high enough to generate heat as the current passes through it. The heating means is electrically connected to the electronics and control module and configured so as to be in the path of the airflow such that, as the airflow comes in contact with the heater means, heat energy is transferred to the airflow, thereby warming the air.

### The Fan (see, generally, 120 and 284)

Embodiments of the present invention further comprise a means for creating an airflow through the unit. Specifically, the airflow is defined as the flow of air entering the unit through the unit inlet and exiting the unit through the unit outlet. Embodiments may have one or more than one inlet and one or more than one outlet.

Preferred embodiments of the means for creating an airflow through the unit comprise a fan that is capable of producing an airflow through the various components of the unit and that is electrically connected to the electronics and control module.

### The Electronics and Control Module (see, generally, 110 and 288)

Embodiments of the present invention comprise an electronics and control module. The various electrical components, such as UV LED's, heater, and fan, are electrically connected to the electronics and control module. The electronics and control module may further comprise one or more of the following, as may be known in the art: power input means; power regulation means; processing means; display means; user input means; temperature sensing and reporting means; timer means; and UV radiation sensing and reporting means.

### The Housing 150

Embodiments of the present invention further comprise a housing. The housing encloses and locates the other components and protects the user from exposure to the internal components and has a one or more inlet opening coupled to the one or more input to the one or more airflow and irradiation management chambers and a one or more outlet opening coupled to the one or more airflow output from the unit.

### The Harware Kit

Embodiments of the present invention may further comprise a hardware kit (not shown). The hardware kit may further comprise mechanical and electrical components. The mechanical components may include, but are not limited to, screws, nuts, bolts, washers, seals, gaskets, caps, and connectors. The electrical components may include, but are not limited to, cables, wire harnesses, electrical connectors, switches, wirenuts, circuit boards, circuit breakers, and fuses.

### The System

Embodiments of the present invention may comprise a system for providing clean, thermally-controlled air to patients for the prevention of hypothermia as might occur intraoperatively or postoperatively. Embodiments of the system may comprise: a particulate filter apparatus; a UV LED air sterilization apparatus capable of achieving at least a 2 LOG kill of airborne pathogens; a heater apparatus; a blower apparatus; an electronics and control apparatus; a flexible hose apparatus; and an inflatable thermal patient covering apparatus.

### The Method (not part of the invention)

The present disclosure includes method steps integral to the use and operation of the disclosed apparatus and system. Embodiments of the related method for providing clean, thermally-controlled air to patients for the prevention of hypothermia as might occur intraoperatively or postoperatively, may comprise the steps of: providing a patient temperature control system comprising; a high performance particulate filter or HEPA filter apparatus; a UV LED air sterilization apparatus capable of achieving at least 2 LOG kill of airborne pathogens; a heater apparatus; a blower apparatus; an electronics and control apparatus; a flexible hose apparatus; and an inflatable thermal patient covering apparatus; then drawing an ambient air flow through the particulate filter apparatus; exposing the ambient air flow to a UV radiation within the UV LED air sterilization apparatus; heating the ambient air flow with the heater apparatus; forcing the ambient air flow, now heated and sterilized, through the flexible hose apparatus; inflating the thermal patient covering apparatus with the now heated and sterilized ambient air flow; and expelling the now heated and sterilized ambient air flow from the inflatable patient covering apparatus to the patient, whereby the apparatus is used to deliver clean, pure air to the patient for clean and effective management of patient body temperature.

In light of the foregoing description, it should be recognized that embodiments in accordance with the present invention can be realized in numerous configurations contemplated to be within the scope of the claims. Additionally, the description above is intended by way of example only and is not intended to limit the present invention in any way, except as set forth in the following claims.

## Claims

1. A system for providing clean, thermally-controlled air to a patient for the prevention of hypothermia, the system comprising:
a particulate filter apparatus (145, 270);
a UV LED air sterilization apparatus (100, 200) that includes an airflow and irradiation management chamber (140, 210);
a heater apparatus (130,286);
a blower apparatus;
an electronics and control module (110, 288);
a flexible hose apparatus; and
an inflatable thermal patient covering apparatus,
wherein the system provides the clean, thermally-controlled air to the patient by:
drawing air through the particulate filter apparatus (145, 270);
exposing the air to UV radiation in the UV LED air sterilization apparatus (100, 200), wherein a plurality of UV LEDs (310) are electrically connected to the electronics and control module (110, 288) and fixedly attached to a plurality of mated openings (261, 461) longitudinally disposed in the wall of the airflow and irradiation management chamber such that the plurality of UV LEDs irradiate the inner surface (240, 450) of the airflow and irradiation management chamber without impeding airflow through the airflow and irradiation management chamber;
heating the air in the heating apparatus (130, 286); and
expelling the heated and exposed air by the blower apparatus through the flexible hose apparatus into the inflatable thermal patient covering apparatus,
wherein the airflow and irradiation management chamber comprises one or more turbulators (320) configured to create one or more area of turbulent airflow.

2. The system of claim 1, wherein the one or more area of turbulent airflow modulates the airflow being irradiated by the UV LEDs (310), wherein the airborne pathogens are exposed to the UV radiation for a duration greater than one second before exiting the outlet of the airflow and irradiation management chamber (140, 210).

3. The system of claim 1, wherein the particulate filter apparatus (145, 270) comprises at least one high performance particulate filter.

4. The system of claim 1, further comprising a safety interlock to shut off the UV LEDs (310) if a part of the system is opened such that the UV LEDs are exposed.

5. The system of claim 1, further comprising a timer means to indicate when to replace the filter.

6. The system of claim 1, wherein an inner surface (240, 450) of the UV LED air sterilization apparatus (100, 200) is treated with a diffuse UV reflector, wherein the diffuse UV reflector comprises at least one of a micro-texture, a coating, or a laminate material.

7. The system of claim 1, further comprising a power supply and regulator configured to provide power to, monitor, and regulate the UV LEDs.

8. The system of claim 1, wherein the one or more turbulators (320) are located within the hollow section of the airflow and irradiation management chamber and disrupt the airflow, by changing laminar airflow (401) into a turbulent airflow (321).

9. The system of claim 1 or 8, wherein the form of the one or more turbulators (320) is selected from the group consisting of turbine vanes, airfoils, v-gutters, grooves, ridges and baffles.

## Patentansprüche

1. System zum Bereitstellen von sauberer, thermisch gesteuerter Luft für einen Patienten zur Verhinderung von Hypothermie, wobei das System Folgendes umfasst:
eine Partikelfiltervorrichtung (145, 270);
eine UV-LED-Luftsterilisationsvorrichtung (100, 200), die eine Luftstrom- und Bestrahlungsbehandlungskammer (140, 210) beinhaltet;
eine Heizvorrichtung (130, 286);
eine Gebläsevorrichtung;
ein Elektronik- und Steuermodul (110, 288);
eine flexible Schlauchvorrichtung; und
eine aufblasbare thermische Patientenabdeckvorrichtung,
wobei das System die saubere, thermisch gesteuerte Luft für den Patienten bereitstellt durch:
Ansaugen von Luft durch die Partikelfiltervorrichtung (145, 270);
Belichten der Luft mit UV-Strahlung in der UV-LED-Luftsterilisationsvorrichtung (100, 200), wobei eine Vielzahl von UV-LED (310) elektrisch mit dem Elektronik- und Steuermodul (110, 288) verbunden und fest an einer Vielzahl von miteinander verbundenen Öffnungen (261, 461) befestigt ist, die in Längsrichtung in der Wand der Luftstrom- und Bestrahlungsbehandlungskammer angeordnet sind, sodass die Vielzahl von UV-LED die Innenfläche (240, 450) der Luftstrom- und Bestrahlungsbehandlungskammer bestrahlt, ohne den Luftstrom durch die Luftstrom- und Bestrahlungsbehandlungskammer zu beeinträchtigen;
Erwärmen der Luft in der Heizvorrichtung (130, 286); und
Ausstoßen der erwärmten und belichteten Luft durch die Gebläsevorrichtung durch die flexible Schlauchvorrichtung hindurch in die aufblasbare thermische Patientenabdeckvorrichtung,
wobei
die Luftstrom- und Bestrahlungsbehandlungskammer einen oder mehrere Turbulatoren (320) umfasst, die dazu konfiguriert sind, einen oder mehrere Bereiche eines wirbelnden Luftstroms zu schaffen.

2. System nach Anspruch 1, wobei der eine oder die mehreren Bereiche eines wirbelnden Luftstroms den Luftstrom modulieren, der durch die UV-LED (310) bestrahlt wird, wobei die durch Luft übertragenen Erreger der UV-Strahlung für eine Dauer von über einer Sekunde ausgesetzt sind, bevor sie aus dem Auslass der Luftstrom- und Bestrahlungsbehandlungskammer (140, 210) austreten.

3. System nach Anspruch 1, wobei die Partikelfiltervorrichtung (145, 270) mindestens einen Hochleistungs-Partikelfilter umfasst.

4. System nach Anspruch 1, ferner umfassend eine Sicherheitsverriegelung zum Abschalten der UV-LED (310), wenn ein Teil des Systems geöffnet wird, sodass die UV-LED freigelegt sind.

5. System nach Anspruch 1, ferner umfassend eine Zeitgebereinrichtung, um anzugeben, wann der Filter zu ersetzen ist.

6. System nach Anspruch 1, wobei eine Innenfläche (240, 450) der UV-LED-Luftsterilisationsvorrichtung (100, 200) mit einem diffusen UV-Reflektor behandelt ist, wobei der diffuse UV-Reflektor mindestens eines von einer Mikrotextur, einer Beschichtung oder einem Laminatmaterial umfasst.

7. System nach Anspruch 1, ferner umfassend eine Leistungsversorgung und einen Regler, die dazu konfiguriert sind, den UV-LED Leistung bereitzustellen, diese zu überwachen und zu regulieren.

8. System nach Anspruch 1, wobei sich der eine oder die mehreren Turbulatoren (320) innerhalb des hohlen Abschnitts der Luft- und Bestrahlungsbehandlungskammer befinden und den Luftstrom stören, indem ein geschichteter Luftstrom (401) in einen wirbelnden Luftstrom (321) umgewandelt wird.

9. System nach Anspruch 1 oder 8, wobei die Form des einen oder der mehreren Turbulatoren (320) aus der Gruppe ausgewählt ist, die aus Turbinenschaufeln, Flügeln, V-Rinnen, Nuten, Stegen und Leitblechen besteht.

## Revendications

1. Système de fourniture d'air propre thermorégulé à un patient pour la prévention de l'hypothermie, le système comprenant :
un appareil de filtration de particules (145, 270) ;
un appareil de stérilisation de l'air par LED UV (100, 200) qui comporte une chambre de gestion de flux d'air et d'irradiation (140, 210) ;
un appareil de chauffage (130, 286) ;
un appareil de soufflage ;
un module électronique et de commande (110, 288) ;
un appareil à tuyau flexible ; et
un appareil de couverture thermique à destination de patients, dans lequel le système fournit l'air propre thermorégulé au patient par :
aspiration d'air à travers l'appareil de filtration de particules (145, 270) ;
exposition de l'air à un rayonnement UV dans l'appareil de stérilisation de l'air par LED UV (100, 200), dans lequel une pluralité de LED UV (310) sont électriquement connectées au module électronique et de commande (110, 288) et fixées de manière immobile à une pluralité d'ouvertures appariées (261, 461) agencées longitudinalement dans la paroi de la chambre de gestion de flux d'air et d'irradiation de telle sorte que la pluralité de LED UV irradient la surface interne (240, 450) de la chambre de gestion de flux d'air et d'irradiation sans entraver le flux d'air dans la chambre de gestion du flux d'air et d'irradiation ;
chauffage de l'air dans l'appareil de chauffage (130, 286) ; et expulsion de l'air chauffé et exposé par l'appareil de soufflage à travers l'appareil à tuyau flexible dans l'appareil de couverture thermique gonflable à destination de patients,
dans lequel
la chambre de gestion de flux d'air et d'irradiation comprend un ou plusieurs turbulateurs (320) configurés pour créer une ou plusieurs zones de flux d'air turbulent.

2. Système selon la revendication 1, dans lequel l'une ou plusieurs zones de turbulence du flux d'air modulent le flux d'air irradié par les LED UV (310), dans lequel les agents pathogènes aériens sont exposés au rayonnement UV pendant une durée supérieure à une seconde avant de sortir par la sortie de la chambre de gestion du flux d'air et d'irradiation (140, 210).

3. Système selon la revendication 1, dans lequel l'appareil de filtration de particules (145, 270) comprend au moins un filtre à particules haute performance.

4. Système selon la revendication 1, comprenant en outre un verrouillage de sécurité pour arrêter les LED UV (310) si une partie du système est ouverte de telle sorte que les LED UV sont exposées.

5. Système selon la revendication 1, comprenant en outre une minuterie pour indiquer quand remplacer le filtre.

6. Système selon la revendication 1, dans lequel une surface interne (240, 450) de l'appareil de stérilisation de l'air par LED UV (100, 200) est traitée avec un réflecteur UV diffus, dans lequel le réflecteur UV diffus comprend au moins l'un d'une microtexture, d'un revêtement ou d'un matériau stratifié.

7. Système selon la revendication 1, comprenant en outre une alimentation et un régulateur configurés pour alimenter, surveiller et réguler les LED UV.

8. Système la revendication 1, dans lequel l'un ou plusieurs turbulateurs (320) sont situés dans la section creuse de la chambre de gestion du flux d'air et d'irradiation et perturbent le flux d'air, en transformant le flux d'air laminaire (401) en un flux d'air turbulent (321).

9. Système selon la revendication 1 ou 8, dans lequel l'un ou plusieurs turbulateurs (320) sont choisis dans le groupe constitué d'aubes de turbine, de pales, de gouttières en V, de rainures, d'arêtes et de chicanes.
